# EUROPEAN PATENT APPLICATION

(11) **EP 1 890 141 A1**
(43) Date of publication of application: **20.02.2008**
(21) Application number: 05811708.6
(22) Date of filing: 29.11.2005
(51) Int. Cl.: G01N 31/00, G01N 27/04, G01N 33/18

(54) **MEASURING METHOD FOR TOTAL ORGANIC CARBON, MEASURING METHOD FOR TOTAL NITROGEN AND MEASURING APPARATUS FOR THE METHODS**

(30) Priority: 26.05.2005 JP 2005154513
(71) Applicant: Shimadzu Corporation, Kyoto-shi, Kyoto 604-8511 (JP)
(72) Inventor: FUJIYAMA, Yoichi, c/o SHIMADZU CORPORATION, Kyoto-shi, Kyoto 604-8511 (JP); MORITA, Yozo, c/o SHIMADZU CORPORATION, Kyoto-shi, Kyoto 604-8511 (JP); KATASHO, Isao, c/o SHIMADZU CORPORATION, Kyoto-shi, Kyoto 604-8511 (JP)
(74) Representative: Schoppe, Fritz
(86) International application number: PCT/JP2005/021837
(87) International publication number: WO 2006/126296

(57) **Abstract**

Disclosed herein is a method for measuring total organic carbon without the influence of nitrogen components. The method for measuring total organic carbon includes the steps of: (1) adjusting the hydrogen ion concentration of sample water to between pH 4 and pH 6; (2) decomposing organic matter and nitrogen compounds contained in the sample water into carbon dioxide and nitrous acid, respectively, through oxidation and; (3) making the carbon dioxide permeate into measuring water via a gas-permeable part 5a; (4) and measuring the electric conductivity of the measuring water to determine the total organic carbon content of the sample water.

## Description

### TECHNICAL FIELD

The present invention relates to a method for measuring the amount of organic carbon and/or nitrogen contained in sample water and to a measuring apparatus to be used for such a measuring method. More particularly, the present invention relates to a method for measuring the total organic carbon content or the total nitrogen content of water containing few impurities, called pure water or ultrapure water, by decomposing organic substances and nitrogen components contained in sample water through oxidation, separating them from the sample water with the use of a gas-permeable part, and measuring electric conductivity, and to a measuring apparatus to be used for such a measuring method.

### BACKGROUND ART

In Japan, methods for measuring organic pollutants, such as BOD (Biochemical Oxygen Demand) components and COD (Chemical Oxygen Demand) components as well as nitrogen compounds and phosphorous compounds contained in water are standardized by Japanese Industrial Standards (JIS). For the measuring of such components in water, an ultraviolet spectrophotometric method (see Nonpatent Document 1) is most widely used for its simple mechanism.

In the case of using the ultraviolet spectrophotometric method, potassium peroxydisulfate is added as an oxidizing agent to sample water, and nitrogen compounds contained in the sample water are thermally decomposed into nitrite ions under high-temperature and high-pressure. Then, the hydrogen ion concentration of the sample water is adjusted to between pH 2 and pH 3, and the absorbance of the sample water is measured at a wavelength of 220 nm to determine the total nitrogen content of the sample water.

The total organic carbon (TOC) content of sample water is measured in the following manner. Potassium peroxydisulfate is added to sample water, and the sample water is heated. Then, organic matter contained in the sample water is decomposed through oxidation by, for example, irradiating the sample water with UV light Only a gas component contained in the sample water is made to permeate into measuring water, which is separated from the sample water by a gas-permeable membrane, to eliminate the influence of various substances contained in the sample water on measurement Then, the electric conductivity of the measuring water is measured to determine the total organic carbon content of the sample water based on a change in the electric conductivity of the measuring water (see Patent Document 1).

When carbonic acid components (carbonic acid, hydrogen carbonate ions, carbonate ions) generated by oxidative decomposition of organic matter contained in sample water are made to permeate into measuring water, an inorganic acid such as phosphoric acid or sulfuric acid is generally added to the sample water to make the sample water acidic. However, the hydrogen ion concentration of the sample water is not particularly controlled, and therefore permeation of the carbonic acid components into measuring water is usually carried out under relatively strong acidic conditions (i.e., at pH 3 or less).
Nonpatent Document 1: JIS K0102 45.2
Patent Document 1: Japanese Patent No. 2510368
Patent Document 2: US Patent No. 5132094
Patent Document 3: Japanese Patent Publication No. Hei 4-507141
Patent Document 4: Japanese Patent Application Laid-open No. Hei 5-034336

### DISCLOSURE OF THE INVENTION

### PROBLEMS TO BE SOLVED BY THE INVENTION

When sample water containing nitrogen components is oxidized by UV light, nitrous acid is generated. In this case, there is a problem in that when the sample water is made acidic to pH 3 or less, nitric oxide generated by decomposition of the nitrous acid is transferred into measuring water and then influences the electric conductivity of the measuring water, which interferes with the measurement of total organic carbon content of the sample water.
In order to eliminate the influence of nitrous acid on measurement of TOC, the use of a gas-permeable part which is selectively permeable to carbon dioxide has been proposed (see Patent Document 2). However, this involves problems that the choice of materials of the gas-permeable part having gas selectivity is limited, the gas transmission rate of the gas-permeable part is slow, and the gas-permeable part has limitations on its gas selectivity.

It is therefore an object of the present invention to provide a method for measuring total organic carbon without the influence of nitrogen components, and a measuring apparatus to be used for such a measuring method.

### MEANS FOR SOLVING THE PROBLEMS

In order to achieve the above object, the present invention is directed to a method for measuring total organic carbon, including the steps of: decomposing organic matter contained in sample water into carbon dioxide through oxidation; making the carbon dioxide to permeate into deionized measuring water via a gas-permeable part; and measuring the electric conductivity of the measuring water to determine the total organic carbon content of the sample water, wherein the hydrogen ion concentration of the sample water is adjusted to between pH 4 and pH 6.
Examples of the gas-permeable part used in the present invention include, but are not limited to, gas-permeable membranes. It is essential as long as the sample water and the measuring water (i.e., deionized water) are separated as liquid phases from each other in such a manner that a gas (usually air) can flow between them. For example, the sample water and the measuring water may be separated from each other without providing a membrane between them as long as a gas can flow between them. The same goes for a second gas-permeable part (which will be described later).
The adjustment of the hydrogen ion concentration of the sample water is made before or after oxidative decomposition. It is essential as long as the adjustment of the hydrogen ion concentration of the sample water is made before the step of making carbon dioxide permeate into measuring water via the gas-permeable part

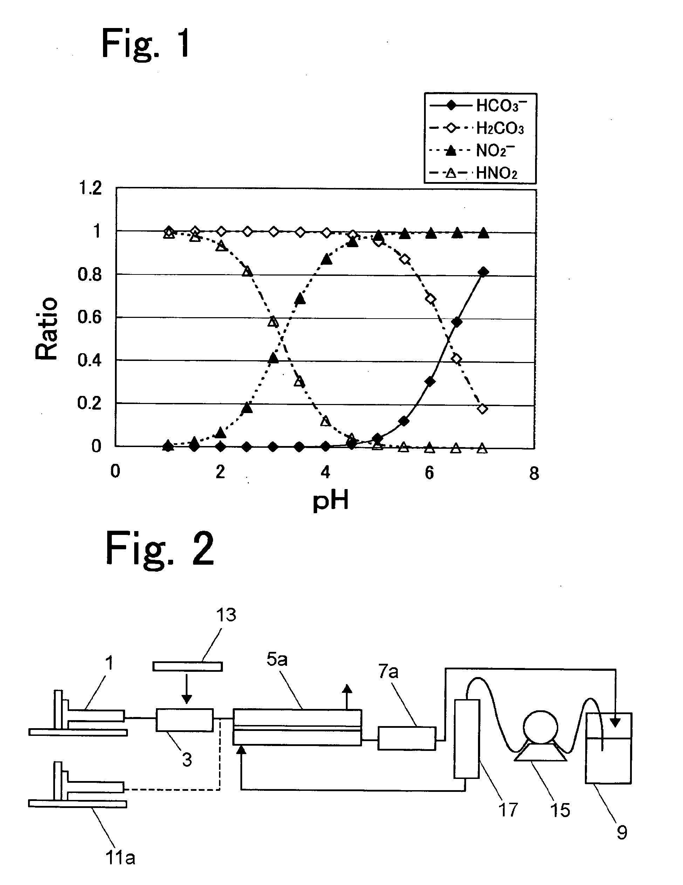

Fig. 1 is a graph which shows changes in the ratio between HCO₃⁻ and H₂CO₃ present in water and in the ratio between NO₂⁻ and HNO₂ present in water when the hydrogen ion concentration of the water is changed from pH 1 to 7. As shown in Fig. 1, most of the carbonic acid is dissolved in water in the form of H₂CO₃ under more acidic conditions than pH 6 to pH 7, but is present in the form of hydrogen carbonate ion (HCO₃⁻) under more basic conditions than pH 6 to 7. On the other hand, most of the nitrous acid is present in the form of HNO₂ under more acidic conditions than pH 3 to 4, but is present in the form of nitrite ion (NO₂⁻) under more basic conditions than pH 3 to pH 4.
Carbonic acid and nitrous acid are easily decomposed and produce carbon dioxide (CO₂) and nitric oxide (NO) respectively, but hydrogen carbonate ions and nitrite ions do not produce such gases.
Therefore, by acidifying the sample water so that the pH of the sample water lies within the range of 4 to 6, it is possible to allow nitrous acid in the sample water to be present in the form of nitrite ion and therefore to prevent the generation of nitric oxide from nitrous acid and the transfer of the nitric oxide into the measuring water. This makes it possible to eliminate the influence of nitrous acid on measurement of carbonic acid.

In a case where the sample water contains nitrogen compounds, the oxidative decomposition step may be performed so that the nitrogen compounds will also be decomposed into nitrous acid through oxidation. In this case, after the organic carbon contained in the sample water is made to permeate into measuring water, the hydrogen ion concentration of the sample water is adjusted to pH 3 or less, the nitrous acid contained in the sample water is made to permeate into another deionized measuring water via another gas-permeable part, and the electric conductivity of the measuring water is measured. In this way, the total nitrogen content of the sample water can also be determined.

By lowering the pH of the sample water to pH 3 or less, it is possible to transform nitrite ions into nitrous acid (nitric oxide) and therefore to separate the nitrous acid from the sample water in gaseous form. Thus, in a case where the sample water contains organic matter and nitrogen compounds, the total nitrogen content of the sample water may also be determined in the following manner. The sample water is treated so that organic matter and nitrogen compounds contained therein will be decomposed into carbon dioxide and nitrous acid, respectively, through oxidation and the hydrogen ion concentration of the sample water is adjusted to between pH 4 and pH 6. Then, the sample water is brought into contact with deionized water via a gas-permeable part to remove carbon dioxide, which has been generated by the decomposition of carbonic acid, from the sample water. Then, the hydrogen ion concentration of the sample water is adjusted to pH 3 or less, and the sample water is brought into contact with another deionized measuring water via another gas-permeable part The electric conductivity of the measuring water is measured to determine the total nitrogen content of the sample water.

Examples of an acid to be added to the sample water for adjusting the hydrogen ion concentration of the sample water include several-normality phosphoric acid conventionally used, diluted acids, and phosphate buffer solutions. However, in the case of adjusting the hydrogen ion concentration of the sample water with a dilute acid to a pH within a predetermined range, it is necessary to accurately control the amount of the dilute acid to be added. In addition, when the sample water is tap water, there is a case where hardness components, such as calcium bicarbonate and the like, dissolved in tap water consume the acid added thereto so that the hydrogen ion concentration of the tap water goes out of the predetermined pH range. For this reason, a pH buffer solution is preferably used for adjusting the hydrogen ion concentration of the sample water.

The present invention is also directed to an apparatus for measuring total organic carbon including: an oxidative decomposition part for decomposing organic matter contained in sample water into carbon dioxide through oxidation; a first adjusting part for adjusting the hydrogen ion concentration of the sample water to between pH 4 and pH 6; a first gas separation part for making the carbon dioxide, which is contained in the sample water having passed through the first adjusting part and the oxidative decomposition part, permeate into deionized measuring water via a gas-permeable part; a first measuring part for measuring the electric conductivity of the measuring water, and an arithmetic part for determining the total organic carbon content of the sample water based on the electric conductivity measured by the first measuring part

In the apparatus for measuring total organic carbon according to the present invention, the oxidative decomposition part may also decompose nitrogen compounds contained in the sample water into nitrite ions through oxidation. In this case, the apparatus for measuring total organic carbon may further include a second adjusting part for adjusting the hydrogen ion concentration of the sample water, which has passed through the first gas separation part, to pH 3 or less; a second gas separation part for making the nitrous acid contained in the sample water, which has passed through the second adjusting part, permeate as nitric oxide into second deionized measuring water via a second gas-permeable part; and a second measuring part for measuring the electric conductivity of the second measuring water containing nitrite ions generated by dissolving the nitric oxide thereinto, wherein the arithmetic part determines also the total nitrogen content of the sample water based on the electric conductivity measured by the second measuring part

The present invention is also directed to an apparatus for measuring total nitrogen, including an oxidative decomposition part for decomposing organic matter and nitrogen compounds contained in sample water into carbon dioxide and nitrous acid respectively, through oxidation; a first adjusting part for adjusting the hydrogen ion concentration of the sample water between pH 4 and pH 6; a first gas separation part for removing the carbon dioxide from the sample water, which has passed through the first adjusting part and the oxidative decomposition part, by bringing the sample water into contact with deionized water via a gas-permeable part; a second adjusting part for adjusting the hydrogen ion concentration of the sample water, which has passed through the first gas separation part, to pH 3 or less; a second gas separation part for bringing the nitrous acid into contact with second measuring water via a second gas-permeable part; a measuring part for measuring the electric conductivity of the measuring water, and an arithmetic part for determining the total nitrogen content of the sample water based on the electric conductivity measured by the measuring part

### Effect of the invention

The method and apparatus for measuring total organic carbon according to the present invention enables measuring the total organic carbon content of sample water without the influence of nitrogen components by adjusting the hydrogen ion concentration of the sample water between pH 4 and pH 6.

In a case where the sample water contains nitrogen compounds, the total nitrogen content of the sample water can be measured by adjusting the hydrogen ion concentration of the sample water to pH 3 or less to transform the nitrogen compounds contained in measuring water into nitrous acid after carbon dioxide contained in the sample water is made to permeate into measuring water.

The method and apparatus for measuring total nitrogen according to the present invention enables measuring the total nitrogen content of sample water by adjusting the hydrogen ion concentration of sample water to between pH 4 and pH 6 to remove carbon dioxide from the sample water, adjusting the hydrogen ion concentration of the sample water to pH 3 or less to transform nitrite ions into nitrous acid, and measuring the electric conductivity of measuring water containing the nitrous acid.

The hydrogen ion concentration of the sample water can be adjusted using a pH buffer solution, which enables providing a measuring environment where large pH fluctuations do not occur.

### BRIEF DESCRIPTION OF THE DRAWINGS

Fig. 1 is a graph which shows changes in the ratio between HCO₃⁻ and H₂CO₃ present in water and in the ratio between NO₂⁻ and HNO₂ present in water when the hydrogen ion concentration of the water is changed from pH 1 to 7;
Fig. 2 is a schematic diagram which shows the flow path of an embodiment of a measuring apparatus for total organic carbon according to the present invention; and
Fig. 3 is a schematic diagram which shows the flow path of another embodiment of the measuring apparatus for total organic carbon according to the present invention combined with a measuring apparatus for total nitrogen.

### EXPLANATION OF REFERENCE NUMERALS

- 1: Syringe pump
- 3: Oxidative decomposition part
- 5a, 5b: Gas separation part
- 7a, 7b: Electric conductivity measuring part
- 9: Fluid reservoir
- 11a, 11b: pH adjusting part
- 13: UV lamp
- 15: Pump
- 17: Ion-exchange resin

### BEST MODE FOR CARRYING OUT THE INVENTION

First, a general method for measuring total organic carbon will be described.
The measuring method includes the steps of: (1) decomposing organic matter contained in sample water into carbon dioxide through oxidation; (2) making the carbon dioxide permeate into measuring water via a gas-permeable part; and (3) measuring the electric conductivity of the measuring water to determine the total organic carbon content of the sample water.

Using this method, the total organic carbon content of sample water is measured. The sample water is an aqueous urea solution (TOC concentration: 0.5 mg/L) corresponding to an aqueous solution of potassium biphthalate (TOC concentration: 0.5 mg/L) which is a reference material for measurement of TOC. TOC measurement is carried out in two cases: one where 6 N phosphoric acid (pH: about 1) is added to the sample water in a volume ratio of 1:200, and the other where a 1 N sodium dihydrogen phosphate solution (pH: about 5) is added to the sample water in a volume ratio of 1: 200.

In a case where 6 N phosphoric acid (pH: about 1) is added, a measurement value was 5.71 mg/L, whereas in a case where a 1 N sodium dihydrogen phosphate solution (pH: about 5) is added, a measurement value was 0.49 mg/L
As shown by the following oxidative decomposition reaction formula (1), oxidation of urea (CO(NH₂)₂) produces nitrous acid.

2CO(NH₂)₂ + 6O₂ → 2CO₂ + 2H₂O + 4HNO₂ (1)

From the measurement result, it can be considered that urea is decomposed to generate nitrous acid (nitric oxide), which can pass through a gas-permeable part, under conditions where the hydrogen ion concentration of the sample water is pH 2 or less, and therefore the measurement value of TOC obtained by adding 6 N phosphoric acid contains also the amount of nitrogen.

More specifically, the measurement value of TOC obtained by adding a strong acid (pH: about 1) was several times larger than the actual total organic carbon content of the sample water due to the interference of nitrous acid, whereas the measurement value of TOC obtained under conditions where the hydrogen ion concentration of the sample water was adjusted to about pH 5 was normal. This indicates that it is necessary to adjust the hydrogen ion concentration of sample water to a pH within the range of 4 to 6 before the step (2). When the hydrogen ion concentration of sample water is within the above pH range, nitrogen components contained in the sample water are present in the form of nitrite ion which cannot pass through a gas-permeable part, and therefore it is possible to measure the total organic carbon content of the sample water without the influence of the nitrogen components.

Hereinbelow, a method for measuring total organic carbon and a method for measuring total nitrogen according to the present invention will be described. As sample water, one whose hydrogen ion concentration has been previously adjusted to between pH 4 and pH 6 by adding a 1 N sodium dihydrogen phosphate solution is used.
A first embodiment of the method for measuring total organic carbon according to the present invention makes it possible to measure total organic carbon without the influence of nitrogen components. The measuring method for total organic carbon according to the first embodiment of the present invention includes the steps of: (1) adjusting the hydrogen ion concentration of sample water to between pH 4 and pH 6; (2) decomposing organic matter contained in the sample water into carbon dioxide through oxidation; (3) making the carbon dioxide permeate into measuring water via a gas-permeable part; and (4) measuring the electric conductivity of the measuring water to determine the total organic carbon content of the sample water.

A second embodiment of the method for measuring total organic carbon according to the present invention makes it possible to measure both total organic carbon and total nitrogen. The measuring method for total organic carbon according to the second embodiment of the present invention includes the steps of: (1) adjusting the hydrogen ion concentration of sample water to between pH 4 and pH 6; (2) decomposing organic matter contained in the sample water into carbon dioxide through oxidation and decomposing nitrogen compounds into nitrous acid (which is present in the form of nitrite ion at between pH 4 and pH 6) through oxidation; (3) making the carbon dioxide permeate into first measuring water via a gas-permeable part; (4) measuring the electric conductivity of the first measuring water to determine the total organic carbon content of the sample water, (5) adjusting the hydrogen ion concentration of the sample water, from which the carbon dioxide has been removed, to pH 3 or less (nitrite ions are transformed into nitrous acid at pH 3); (6) making the nitrous acid contained in the sample water permeate as nitric oxide into second measuring water via another gas-permeable part; and (7) measuring the electric conductivity of the second measuring water to determine the total nitrogen content of the sample water.

A first embodiment of the method for measuring total nitrogen according to the present invention makes it possible to measure total nitrogen after organic carbon is removed. The measuring method for total nitrogen according to the first embodiment of the present invention includes the steps of: (1) adjusting the hydrogen ion concentration of sample water to between pH 4 and pH 6; (2) decomposing organic matter contained in the sample water into carbon dioxide through oxidation and decomposing nitrogen compounds into nitrite ions through oxidation; (3) removing the carbon dioxide from the sample water with the use of a gas-permeable part; (4) adjusting the hydrogen ion concentration of the sample water, from which the carbon dioxide has been removed, to pH 3 or less; (5) making the nitrous acid contained in the sample water permeate into measuring water via another gas-permeable part; and (6) measuring the electric conductivity of the measuring water to determine the total nitrogen content of the sample water.

It is to be noted that by using a phosphate buffer solution whose pH is kept in the range of 4 to 6 for adjusting the hydrogen ion concentration of the sample water, it is possible to provide a measurement environment where large pH fluctuations do not occur.

Hereinbelow, an embodiment of an apparatus for measuring total organic carbon according to the present invention will be described with reference to Fig. 2 which schematically shows the flow path of the measuring apparatus.
As shown in Fig. 2, a syringe pump 1 for feeding sample water, an oxidative decomposition part 3, a gas separation part 5a, an electric conductivity measuring part 7a, and a fluid reservoir 9 are arranged in this order from the upstream side of the measuring apparatus (i.e., from the left side in the drawing) and are connected to each other through a flow path.

The oxidative decomposition part 3 has a UV lamp 13 to subject sample water to oxidative decomposition using UV light Alternatively, as shown in Fig. 5 in Patent Document 3, the oxidative decomposition part 3 may have a structure in which a flow path for oxidative decomposition is wound around the periphery of a UV lamp.

The gas separation part 5a is used to separate a gas component from the sample water and to transfer the gas component into measuring water. In the gas separation part 5a, the sample water, which has passed through the oxidative decomposition part 3, and circulating deionized measuring water are placed on opposite sides of a gas-permeable part such as a porous PTFE (polytetrafluoroethylene) membrane. Examples of such a gas-permeable part include gas-permeable membranes used in a measuring apparatus for total organic carbon disclosed in Patent Document 4. The gas separation part 5a has an outlet for discharging the sample water after the completion of permeation of the gas component into the measuring water.

The electric conductivity measuring part 7a has an electric conductivity meter for measuring the electric conductivity of the measuring water containing the gas component dissolved therein. The measuring apparatus for total organic carbon according to the present embodiment uses a parallel plate type electric conductivity meter.

Between the fluid reservoir 9 and the gas separation part 5a, there is a pump 15 and an ion-exchange resin 17. The gas separation part 5a, the ion-exchange resin 17, the pump 15, and the fluid reservoir 9 are connected to each other through a flow path. Therefore, the measuring water stored in the fluid reservoir 9 is fed to the ion-exchange resin 17 by the pump 15 for deionization, and then the deionized measuring water is fed into the gas separation part 5a through its measuring water inlet The measuring water receives the gas component from the sample water in the gas separation part 5a, and then the electric conductivity of the measuring water is measured by the electric conductivity measuring part 7a. Thereafter, the measuring water is again fed into the fluid reservoir 9.

The hydrogen ion concentration of the sample water is previously adjusted to between pH 4 and pH 6, and then the sample water is fed into the measuring apparatus by the syringe pump 1. Then, organic matter contained in the sample water is decomposed by the oxidative decomposition part 3 into carbon dioxide through oxidation, and the carbon dioxide is made to permeate into measuring water in the gas separation part 5a. The measuring water is fed to the electric conductivity measuring part 7a to measure the electric conductivity of the measuring water to determine the total organic carbon content of the sample water.
The measuring water is collected after the measurement of electric conductivity, and is stored in the fluid reservoir 9. As described above, the measuring water is fed to the ion-exchange resin 17 by the pump 15 and deionized. In the measuring apparatus for total organic carbon having such a structure described above, the measuring water can be circulated and used.

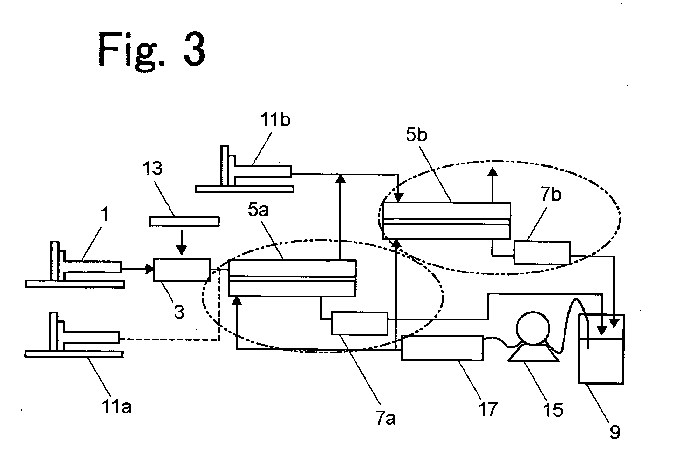

Fig. 3 is a schematic diagram which shows the flow path of an embodiment of a measuring apparatus for total organic carbon and total nitrogen.
The measuring apparatus for total organic carbon and total nitrogen shown in Fig. 3 includes, in addition to the components of the measuring apparatus for total organic carbon shown in Fig. 2, a gas separation part 5b for separating nitrous acid from sample water, an electric conductivity measuring part 7b for measuring the electric conductivity of measuring water containing the nitrous acid, and a pH adjusting part 11 b for transforming nitrite ions into nitrous acid.
The gas separation part 5b is connected to the outlet of the gas separation part 5a, and the pH adjusting part 11 b is connected to the inlet of the gas separation part 5b. A liquid to be used in the pH adjusting part 11 b is not particularly limited as long as it can adjust the hydrogen ion concentration of sample water to pH 3 or less to make the sample water acidic. For example, a phosphate buffer solution can be used.

As described above, the pump 15 and the ion-exchange resin 17 are provided between the fluid reservoir 9 and the measuring water inlet of the gas separation part 5a, and they are connected to each other through a flow path. Likewise, the measuring water inlet of the gas separation part 5b, the ion-exchange resin 17, the pump 15, and the fluid reservoir 19 are also connected to each other through a flow path. Therefore, measuring water stored in the fluid reservoir 9 is fed to the ion-exchange resin 17 by the pump 15 for deionization, and then the deionized measuring water is fed into the gas separation parts 5a and 5b through their respective measuring water inlets. The measuring water fed into the gas separation part 5a receives a gas component from the sample water, and then the electric conductivity of the measuring water is measured by the electric conductivity measuring part 7a. On the other hand, the measuring water fed into the gas separation part 5b also receives another gas component from the sample water, and then the electric conductivity of the measuring water is measured by the electric conductivity measuring part 7b. Thereafter, the measuring water discharged from the electric conductivity measuring parts 7a and 7b is again fed into the fluid reservoir 9.

The hydrogen ion concentration of the sample water is previously adjusted to between pH 4 and pH 6, and then the sample water is fed into the measuring apparatus by the syringe pump 1. Then, organic matter and nitrogen compounds contained in the sample water are decomposed by the oxidative decomposition part 3 into carbon dioxide and nitrous acid, respectively, and only the carbon dioxide is made to permeate into measuring water via the gas separation part 5a (nitrous acid is present in the form of nitrite ion at a pH of 4 to 6). Then, the measuring water is fed to the electric conductivity measuring part 7a to measure the electric conductivity of the measuring water to determine the total organic carbon content of the sample water.
Then, an acid is added to the sample water, from which the carbon dioxide has been removed, by the pH adjusting part 11 b to adjust the hydrogen ion concentration of the sample water to pH 3 or less (nitrite ions are transformed into nitrous acid). This sample water is fed into the gas separation part 5b to make the nitrous acid contained in the sample water permeate as nitric oxide into measuring water. Then, the measuring water is fed into the electric conductivity measuring part 7b to measure the electric conductivity of the measuring water to determine the total nitrogen content of the sample water.
The measuring water discharged from both the electric conductivity measuring parts 7a and 7b is collected and stored in the fluid reservoir 9. Then, the measuring water is fed into the ion-exchange resin 17 by the pump 15 and deionized. In the measuring apparatus for total organic carbon and total nitrogen having such a structure described above, the measuring water can be shared between the measurement of total organic carbon and the measurement of total nitrogen.

Next, another embodiment of the measuring apparatus for total organic carbon and another embodiment of the measuring apparatus for total nitrogen according to the present invention will be described.
In the case of the above-described measuring apparatuses according to the present invention shown in Figs. 2 and 3, sample water whose hydrogen ion concentration has been previously adjusted to between pH 4 and pH 6 is used, but in a case where sample water whose hydrogen ion concentration has not been previously adjusted is used, a pH adjusting part is further provided.
As shown by the broken line in Figs. 2 and 3, a pH adjusting part 11 a can be connected to a flow path between the oxidative decomposition part 3 and the gas separation part 5a. Alternatively, the pH adjusting part 11a may be connected to a flow path between the syringe pump 1 and the oxidative decomposition part 3.
As a pH adjusting agent to be added to sample water, one which can adjust the hydrogen ion concentration of the sample water to between pH 4 and pH 6 is used. For example, a phosphate buffer solution can be used.

An acid is added by the pH adjusting part 11 a to sample water, whose hydrogen ion concentration has not been previously adjusted, to adjust the hydrogen ion concentration of the sample water to between pH 4 and pH 6. Then, the total organic carbon content and the total nitrogen content of the sample water are determined in the same manner as in the embodiment described above.
By providing the pH adjusting part 11 a, it is possible to adjust the hydrogen ion concentration of sample water to between pH 4 and pH 6 before the sample water is fed into the gas separation part 5a, even when the hydrogen ion concentration of the sample water has not been previously adjusted to between pH 4 and pH 6.

A measuring apparatus for total nitrogen can be provided by omitting the electric conductivity measuring part 7a connected to the gas separation part 5a from the measuring apparatus shown in Fig. 3. By doing so, it is possible to measure only the total nitrogen content of sample water.

The present invention is not limited to the above embodiments, and various changes and modifications can be carried out within the scope of the appended claims. For example, the oxidative decomposition part 3 may use, instead of the UV lamp for UV irradiation, an autoclave for thermal decomposition under high-temperature and high-pressure. Further, the pH buffer solution is not limited to a phosphate buffer solution, and another buffer solution such as acetate buffer solution may also be used. It is to be noted that in a case where a pH buffer solution containing organic matter is used, the pH buffer solution is added to sample water after the sample water is discharged from the oxidative composition part

### INDUSTRIAL APPLICABILITY

The measuring method and measuring apparatus for total organic carbon and/or total nitrogen according to the present invention can be used for measuring the amount of organic substances and/or nitrogen components contained in various kinds of sample water such as water containing few impurities, called pure water or ultrapure water.

## Claims

1. A method for measuring total organic carbon, comprising the steps of:
decomposing organic matter contained in sample water into carbon dioxide through oxidation;
making the carbon dioxide to permeate into deionized measuring water via a gas-permeable part; and
then measuring the electric conductivity of the measuring water to determine the total organic carbon content of the sample water,
wherein the hydrogen ion concentration of the sample water is adjusted to between pH 4 and pH 6.

2. The method for measuring total organic carbon according to claim 1, wherein in the step of oxidative decomposition, nitrogen compounds contained in the sample water are also decomposed into nitrous acid through oxidation, the method further comprising the steps in the order named:
adjusting the hydrogen ion concentration of the sample water to pH 3 or less after the carbon dioxide contained in the sample water is made to permeate into the measuring water,
making the nitrous acid contained in the sample water permeate into second deionized measuring water via another gas-permeable part; and
measuring the electric conductivity of the second measuring water to determine also the total nitrogen content of the sample water.

3. The method for measuring total organic carbon according to claim 1 or 2, wherein the hydrogen ion concentration of the sample water is adjusted using a pH buffer solution.

4. A method for measuring total nitrogen, comprising the steps in the order named:
treating the sample water so that organic matter and nitrogen compounds, which may be contained in the sample water, will be decomposed into carbon dioxide and nitrous acid, respectively, through oxidation;
removing the carbon dioxide from the sample water by bringing the sample water, whose hydrogen ion concentration has been adjusted to between pH 4 and pH 6, into contact with deionized water via a gas-permeable part;
adjusting the hydrogen ion concentration of the sample water to pH 3 or less;
bringing the sample water into contact with deionized measuring water via another gas-permeable part; and
measuring the electric conductivity of the measuring water to determine the total nitrogen content of the sample water.

5. The method for measuring total nitrogen according to claim 4, wherein the hydrogen ion concentration of the sample water is adjusted using a pH buffer solution.

6. An apparatus for measuring total organic carbon comprising:
an oxidative decomposition part for decomposing organic matter contained in sample water into carbon dioxide through oxidation;
a first adjusting part for adjusting the hydrogen ion concentration of the sample water to between pH 4 and pH 6;
a first gas separation part for making the carbon dioxide, which is contained in the sample water having passed through the first adjusting part and the oxidative decomposition part, permeate into deionized measuring water via a gas-permeable part;
a first measuring part for measuring the electric conductivity of the measuring water, and
an arithmetic part for determining the total organic carbon content of the sample water based on the electric conductivity measured by the first measuring part

7. The apparatus for measuring total organic carbon according to claim 6, wherein the oxidative decomposition part decomposes also nitrogen compounds contained in the sample water into nitrous acid through oxidation, further comprising;
a second adjusting part for adjusting the hydrogen ion concentration of the sample water, which has passed through the first gas separation part, to pH 3 or less;
a second gas separation part for making the sample water, which has passed through the second adjusting part, permeate into second deionized measuring water via a second gas-permeable part; and
a second measuring part for measuring the electric conductivity of the second measuring water, wherein the arithmetic part determines also the total nitrogen content of the sample water based on the electric conductivity measured by the second measuring part

8. An apparatus for measuring total nitrogen, comprising:
an oxidative decomposition part for decomposing organic matter and nitrogen compounds contained in sample water into carbon dioxide and nitrous acid, respectively, through oxidation;
a first adjusting part for adjusting the hydrogen ion concentration of the sample water to between pH 4 and pH 6;
a first gas separation part for removing the carbon dioxide from the sample water, which has passed through the first adjusting part and the oxidative decomposition part, by bringing the sample water into contact with deionized water via a gas-permeable part;
a second adjusting part for adjusting the hydrogen ion concentration of the sample water, which has passed through the first gas separation part, to pH 3 or less;
a second gas separation part for bringing the sample water, which has passed through the second adjusting part, into contact with second deionized measuring water via a second gas-permeable part;
a measuring part for measuring the electric conductivity of the measuring water, and
an arithmetic part for determining the total nitrogen content of the sample water based on the electric conductivity measured by the measuring part
